# EUROPEAN PATENT SPECIFICATION

(11) **EP 2 824 186 B1**
(45) Date of publication and mention of the grant of the patent: **11.04.2018**
(21) Application number: 14748825.8
(22) Date of filing: 07.01.2014
(51) Int. Cl.: C12P 13/08, C12N 15/70, C12N 1/21, C12N 15/61

(54) **L-LYSINE GENERATION METHOD BY FERMENTING BACTERIA HAVING MODIFIED ACONITASE GENE AND/OR REGULATORY ELEMENT**
VERFAHREN ZUR ERZEUGUNG VON L-LYSIN DURCH FERMENTATIONSBAKTERIEN MIT MODIFIZIERTEM ACONITASEGEN UND REGULATORISCHEM ELEMENT
PROCÉDÉ DE GÉNÉRATION DE L-LYSINE PAR FERMENTATION DE BACTÉRIES DONT LE GÈNE ACONITASE ET/OU L'ÉLÉMENT RÉGULATEUR A ÉTÉ MODIFIÉ

(30) Priority: 08.02.2013 CN 201310050196; 08.02.2013 CN 201310050144
(43) Date of publication of application: 14.01.2015
(73) Proprietor: Ningxia Eppen Biotech Co. Ltd, Ningxia 750100 (CN)
(72) Inventor: MA, Jiyin, Yongning Yinchuan Ningxia 750100 (CN); WEN, Tingyi, Yongning Yinchuan Ningxia 750100 (CN); CHEN, Jinlong, Yongning Yinchuan Ningxia 750100 (CN); LIANG, Yong, Yongning Yinchuan Ningxia 750100 (CN); LIU, Shuwen, Yongning Yinchuan Ningxia 750100 (CN); WEI, Aiying, Yongning Yinchuan Ningxia 750100 (CN); YANG, Lipeng, Yongning Yinchuan Ningxia 750100 (CN); REN, Rui, Yongning Yinchuan Ningxia 750100 (CN); MENG, Gang, Yongning Yinchuan Ningxia 750100 (CN); ZHAO, Chunguang, Yongning Yinchuan Ningxia 750100 (CN); ZHANG, Yun, Yongning Yinchuan Ningxia 750100 (CN); SHANG, Xiuling, Yongning Yinchuan Ningxia 750100 (CN); GUO, Xiaowei, Yongning Yinchuan Ningxia 750100 (CN)
(74) Representative: Carpmaels & Ransford LLP
(86) International application number: PCT/CN2014/070228
(87) International publication number: WO 2014/121669

(56) References cited:
- CN-A- 101 631 871
- CN-A- 102 191 289
- CN-A- 103 131 738
- CN-A- 103 146 772
- US-A- 5 766 925
- US-A1- 2002 072 099
- MITSUHASHI SATOSHI ET AL: "Disruption of malate:quinone oxidoreductase increases L-lysine production by Corynebacterium glutamicum", BIOSCIENCE BIOTECHNOLOGY BIOCHEMISTRY, JAPAN SOCIETY FOR BIOSCIENCE, BIOTECHNOLOGY, AND AGROCHEMISTRY, TOKYO, JAPAN, vol. 70, no. 11, 1 November 2006 (2006-11-01), pages 2803-2806, XP002566301, ISSN: 0916-8451, DOI: 10.1271/BBB.60298 [retrieved on 2006-11-07]
- RUKLISHA ET AL: "l-Valine biosynthesis during batch and fed-batch cultivations of Corynebacterium glutamicum: Relationship between changes in bacterial growth rate and intracellular metabolism", PROCESS BIOCHEMISTRY, ELSEVIER, NL, vol. 42, no. 4, 19 March 2007 (2007-03-19) , pages 634-640, XP005933474, ISSN: 1359-5113, DOI: 10.1016/J.PROCBIO.2006.11.008

### SEQUENCE LISTING

<110> Ningxia EPPEN Biotech Co., Ltd
<120> Method for Producing L-Lysine by Modifying Aconitase Gene and/or Regulatory Elements thereof
<130> P064607EP
<140> EP14748825.8
   <141> 2014-09-26
<150> CN 201310050196.0
   <151> 2013-02-08
<150> CN 201310050144.3
   <151> 2013-02-08
<160> 8
<170> SeqWin2010, version 1.0
<210> 1
   <211> 2676
   <212> DNA
   <213> Escherichia coli
<400> 1
   atgtcgtcaa ccctacgaga agccagtaag gacacgttgc aggccaaaga taaaacttac   60
   cactactaca gcctgccgct tgctgctaaa tcactgggcg atatcacccg tctacccaag  120
   tcactcaaag ttttgctcga aaacctgctg cgctggcagg atggtaactc ggttaccgaa  180
   gaggatatcc acgcgctggc aggatggctg aaaaatgccc atgctgaccg tgaaattgcc  240
   taccgcccgg caagggtgct gatgcaggac tttaccggcg tacctgccgt tgttgatctg  300
   gcggcaatgc gcgaagcggt taaacgcctc ggcggcgata ctgcaaaggt taacccgctc  360
   tcaccggtcg acctggtcat tgaccactcg gtgaccgtcg atcgttttgg tgatgatgag  420
   gcatttgaag aaaacgtacg cctggaaatg gagcgcaacc acgaacgtta tgtgttcctg  480
   aaatggggaa agcaagcgtt cagtcggttt agcgtcgtgc cgccaggcac aggcatttgc  540
   catcaggtta acctcgaata tctcggcaaa gcagtgtgga gtgaattgca ggacggtgaa  600
   tggattgctt atccggatac actcgttggt actgactcgc acaccaccat gatcaacggc  660
   cttggcgtgc tggggtgggg cgttggtggg atcgaagcag aagccgcaat gttaggccag  720
   ccggtttcca tgcttatccc ggatgtagtg ggcttcaaac ttaccggaaa attacgtgaa  780
   ggtattaccg ccacagacct ggttctcact gttacccaaa tgctgcgcaa acatggcgtg  840
   gtggggaaat tcgtcgaatt ttatggtgat ggtctggatt cactaccgtt ggcggatcgc  900
   gccaccattg ccaatatgtc gccagaatat ggtgccacct gtggcttctt cccaatcgat  960
   gctgtaaccc tcgattacat gcgtttaagc gggcgcagcg aagatcaggt cgagttggtc 1020
   gaaaaatatg ccaaagcgca gggcatgtgg cgtaacccgg gcgatgaacc aatttttacc 1080
   agtacgttag aactggatat gaatgacgtt gaagcgagcc tggcagggcc taaacgccca 1140
   caggatcgcg ttgcactgcc cgatgtacca aaagcatttg ccgccagtaa cgaactggaa 1200
   gtgaatgcca cgcataaaga tcgccagccg gtcgattatg ttatgaacgg acatcagtat 1260
   cagttacctg atggcgctgt ggtcattgct gcgataacct cgtgcaccaa cacctctaac 1320
   ccaagtgtgc tgatggccgc aggcttgctg gcgaaaaaag ccgtaactct gggcctcaag 1380
   cggcaaccat gggtcaaagc gtcgctggca ccgggttcga aagtcgtttc tgattatctg 1440
   gcaaaagcga aactgacacc gtatctcgac gaactggggt ttaaccttgt gggatacggt 1500
   tgtaccacct gtattggtaa ctctgggccg ctgcccgatc ctatcgaaac ggcaatcaaa 1560
   aaaagcgatt taaccgtcgg tgcggtgctg tccggcaacc gtaactttga aggccgtatc 1620
   catccgctgg ttaaaactaa ctggctggcc tcgccgccgc tggtggttgc ctatgcgctg 1680
   gcgggaaata tgaatatcaa cctggcttct gagcctatcg gccatgatcg caaaggcgat 1740
   ccggtttatc tgaaagatat ctggccatcg gcacaagaaa ttgcccgtgc ggtagaacaa 1800
   gtctccacag aaatgttccg caaagagtac gcagaagttt ttgaaggcac agcagagtgg 1860
   aagggaatta acgtcacacg atccgatacc tacggttggc aggaggactc aacctatatt 1920
   cgcttatcgc ctttctttga tgaaatgcag gcaacaccag caccagtgga agatattcac 1980
   ggtgcgcgga tcctcgcaat gctgggggat tcagtcacca ctgaccatat ctctccggcg 2040
   ggcagtatta agcccgacag cccagcgggt cgatatctac aaggtcgggg tgttgagcga 2100
   aaagacttta actcctacgg ttcgcggcgt ggtaaccatg aagtgatgat gcgcggcacc 2160
   ttcgccaata ttcgcatccg taatgaaatg gtgcctggcg ttgaaggggg gatgacgcgg 2220
   catttacctg acagcgacgt agtctctatt tatgatgctg cgatgcgcta taagcaggag 2280
   caaacgccgc tggcggtgat tgccgggaaa gagtatggat caggctccag tcgtgactgg 2340
   gcggcaaaag gtccgcgtct gcttggtatt cgtgtggtga ttgccgaatc gtttgaacga 2400
   attcaccgtt cgaatttaat tggcatgggc atcctgccgc tggaatttcc gcaaggcgta 2460
   acgcgtaaaa cgttagggct aaccggggaa gagaagattg atattggcga tctgcaaaac 2520
   ctacaacccg gcgcgacggt tccggtgacg cttacgcgcg cggatggtag ccaggaagtc 2580
   gtaccctgcc gttgtcgtat cgacaccgcg acggagttga cctactacca gaacgacggc 2640
   attttgcatt atgtcattcg taatatgttg aagtaa                           2676
<210> 2
   <211> 2832
   <212> DNA
   <213> Corynebacterium glutamicum
<400> 2
   ttggagctca ctgtgactga aagcaagaac tccttcaatg ctaagagcac ccttgaagtt  60
   ggcgacaagt cctatgacta cttcgccctc tctgcagtgc ctggcatgga gaagctgccg  120
   tactccctca aggttctcgg agagaacctt cttcgtaccg aagacggcgc aaacatcacc  180
   aacgagcaca ttgaggctat cgccaactgg gatgcatctt ccgatccaag catcgaaatc  240
   cagttcaccc cagcccgtgt tctcatgcag gacttcaccg gtgtcccttg tgtagttgac  300
   ctcgcaacca tgcgtgaggc agttgctgca ctcggtggcg accctaacga cgtcaaccca  360
   ctgaacccag ccgagatggt cattgaccac tccgtcatcg tggaggcttt cggccgccca  420
   gatgcactgg ctaagaacgt tgagatcgag tacgagcgca acgaggagcg ttaccagttc  480
   ctgcgttggg gttccgagtc cttctccaac ttccgcgttg ttcctccagg aaccggtatc  540
   gtccaccagg tcaacattga gtacttggct cgcgtcgtct tcgacaacga gggccttgca  600
   tacccagata cctgcatcgg taccgactcc cacaccacca tggaaaacgg cctgggcatc  660
   ctgggctggg gcgttggtgg cattgaggct gaagcagcaa tgctcggcca gccagtgtcc  720
   atgctgatcc ctcgcgttgt tggcttcaag ttgaccggcg agatcccagt aggcgttacc  780
   gcaactgacg ttgtgctgac catcaccgaa atgctgcgcg accacggcgt cgtccagaag  840
   ttcgttgagt tctacggctc cggtgttaag gctgttccac tggctaaccg tgcaaccatc  900
   ggcaacatgt ccccagagtt cggctccacc tgtgcgatgt tcccaatcga cgaggagacc  960
   accaagtacc tgcgcctcac cggccgccca gaagagcagg ttgcactggt cgaggcttac  1020
   gccaaggcgc agggcatgtg gctcgacgag gacaccgttg aagctgagta ctccgagtac  1080
   ctcgagctgg acctgtccac cgttgttcct tccatcgctg gccctaagcg cccacaggac  1140
   cgcatccttc tctccgaggc aaaggagcag ttccgtaagg atctgccaac ctacaccgac  1200
   gacgctgttt ccgtagacac ctccatccct gcaacccgca tggttaacga aggtggcgga  1260
   cagcctgaag gcggcgtcga agctgacaac tacaacgctt cctgggctgg ctccggcgag  1320
   tccttggcta ctggcgcaga aggacgtcct tccaagccag tcaccgttgc atccccacag  1380
   ggtggcgagt acaccatcga ccacggcatg gttgcaattg catccatcac ctcttgcacc  1440
   aacacctcta acccatccgt gatgatcggc gctggcctga tcgcacgtaa ggcagcagaa  1500
   aagggcctca agtccaagcc ttgggttaag accatctgtg caccaggttc ccaggttgtc  1560
   gacggctact accagcgcgc agacctctgg aaggaccttg aggccatggg cttctacctc  1620
   tccggcttcg gctgcaccac ctgtattggt aactccggcc cactgccaga ggaaatctcc  1680
   gctgcgatca acgagcacga cctgaccgca accgcagttt tgtccggtaa ccgtaacttc  1740
   gagggacgta tctcccctga cgttaagatg aactacctgg catccccaat catggtcatt  1800
   gcttacgcaa tcgctggcac catggacttc gacttcgaga acgaagctct tggacaggac  1860
   caggacggca acgacgtctt cctgaaggac atctggcctt ccaccgagga aatcgaagac  1920
   accatccagc aggcaatctc ccgtgagctt tacgaagctg actacgcaga tgtcttcaag  1980
   ggtgacaagc agtggcagga actcgatgtt cctaccggtg acaccttcga gtgggacgag  2040
   aactccacct acatccgcaa ggcaccttac ttcgacggca tgcctgtcga gccagtggca  2100
   gtcaccgaca tccagggcgc acgcgttctg gctaagctcg gcgactctgt caccaccgac  2160
   cacatctccc ctgcttcctc cattaagcca ggtacccctg cagctcagta cttggatgag  2220
   cacggtgtgg aacgccacga ctacaactcc ctgggttcca ggcgtggtaa ccacgaggtc  2280
   atgatgcgcg gcaccttcgc caacatccgc ctccagaacc agctggttga catcgcaggt  2340
   ggctacaccc gcgacttcac ccaggagggt gctccacagg cgttcatcta cgacgcttcc  2400
   gtcaactaca aggctgctgg cattccgctg gtcgtcttgg gcggcaagga gtacggcacc  2460
   ggttcttccc gtgactgggc agctaagggc actaacctgc tcggaattcg cgcagttatc  2520
   accgagtcct tcgagcgtat tcaccgctcc aacctcatcg gtatgggcgt tgtcccactg  2580
   cagttccctg caggcgaatc ccacgagtcc ctgggccttg acggcaccga gaccttcgac  2640
   atcaccggac tgaccgcact caacgagggc gagactccta agactgtcaa ggtcaccgca  2700
   accaaggaga acggcgacgt cgtcgagttc gacgcagttg tccgcatcga caccccaggt  2760
   gaggctgact actaccgcca cggcggcatc ctgcagtacg tgctgcgtca gatggctgct  2820
   tcttctaagt aa                                                     2832
<210> 3
   <211> 161
   <212> DNA
   <213> Escherichia coli
<400> 3
   caatccgacg tctaagaaac cattattatc atgacattaa cctataaaaa taggcgtatc  60
   acgaggccct ttcgtcttca cctcgagtcc ctatcagtga tagagatgga catccctatc  120
   agtgatagag atactgagca catcagcagg acgcactgac c                      161
<210> 4
   <211> 454
   <212> DNA
   <213> Escherichia coli
<400> 4
   ggaatgttcc gtcgttattc cagacgactg gcaactaaca tcgcagcagc aagcctttat  60
   agaactgttt gctgaagatg atcagccgaa acaataatta tcatcattct tattacccat  120
   ttttaatgaa ttaaagggct tttaatacac cgcagcaata acagcttgag ttatctcaac  180
   acaaaataat aaccgttaag ggtgtagcct atgatcaaca caaatatgaa atattggtcc  240
   tggatgggcg cgttttctct gtcgatgctc ttctgggccg aactcctctg gatcattact  300
   cactgatcct tgaccccgct gcggcggggt tgtcatttgc tttgccacaa ggtttctcct  360
   cttttatcaa tttgggttgt tatcaaatcg ttacgcgatg tttgtgttat ctttaatatt  420
   caccctgaag agaatcaggg cttcgcaacc ctgt                              454
<210> 5
   <211> 130
   <212> DNA
   <213> Corynebacterium glutamicum
<400> 5
   ccgttgaaaa ctaaaaagct gggaaggtga atcgaatttc ggggctttaa agcaaaaatg  60
   aacagcttgg tctatagtgg ctaggtaccc tttttgtttt ggacacatgt agggtggccg  120
   aaacaaagta                                                         130
<210> 6
   <211> 166
   <212> DNA
   <213> Corynebacterium glutamicum
<400> 6
   acgcgccaag aaccccaact ttcccgccag aacgcttgta ctgttaggat aatgaagacg  60
   tagggtcctt ttccacagtt ctgtggaatg agaatccgat gtttttctca cgccggctca  120
   gccgaagcag acgccgtcgc gaaatctcac cctaaaaaag ttagaa                 166
<210> 7
   <211> 567
   <212> DNA
   <213> Corynebacterium glutamicum
<400> 7
   gtgtccgtag cggcaggcga caaaccaaca aatagccgtc aagaaatcct cgaaggtgcc  60
   cgacggtgct tcgctgagca cggctatgaa ggcgcaaccg tacgccgact ggaagaagca  120
   acaggtaaat cacgcggagc gatctttcat cacttcggtg acaaagaaaa cctgttccta  180
   gccctcgcgc gggaagatgc agcccgcatg gcggaggtgg tgtctgaaaa tggcctcgtt  240
   gaagtgatgc gaggaatgct ggaagatcct gaacgatatg actggatgtc agtacgcctg  300
   gagatctcca agcagctgcg caccgacccg gtattccgcg caaaatggat tgatcaccaa  360
   agtgttctag acgaagctgt ccgcgtgcgt ttgtcccgca acgtggataa gggacaaatg  420
   cgcactgacg tcccgatcga agtgctgcac accttcttag agactgttct cgacggtttc  480
   atctcccgtc ttgctaccgg cgcatccaca gaaggactgt ccgaagtatt ggatctggtc  540
   gagggaactg tccgtaaacg cgactaa                                      567
<210> 8
   <211> 164
   <212> DNA
   <213> Corynebacterium glutamicum
<400> 8
   ctgcataatt cgtgtcgctc aaggcgcact cccgttctgg ataatgtttt ttgcgccgac  60
   atcataacgg ttctggcaaa tattctgaaa tgagctgttg acaattaatc atcggctcgt  120
   ataatgtgtg gaattgtgag cggataacaa tttcacacag gaaa                   164

## Description

### Technical Field

The present invention belongs to the field of amino acid fermentation, and specifically relates to a method of producing L-lysine by fermentation and methods and uses derived from the method as well as bacteria used in these methods and uses.

### Background Art

To produce L-lysine by fermentation with L-lysine-producing bacteria (e.g., *E. coli* of *Escherichia* and bacteria of *Corynebacterium*) has been already widely industrially used. These bacteria may be isolated from the natural environment, and/or may be obtained by mutagenesis or genetically engineered modification. Prior arts focus on the genetically engineered modifications of genes such as *pnt*, *dap*, *ppc* and the like, and pay no attention to a regulatory element of a gene encoding an aconitase (e.g., aconitase A from *Escherichia coli,* an aconitase from *Corynebacterium glutamicum* or *Corynebacterium pekinense*) for the production of L-lysine. For example, Mitsuhashi *et al.* 2006 discusses disrupting malate:quinone oxidoreductase, US5766925 discusses modifying the gene encoding aspartokinase and US 2002/0072099 discusses the genes encoding *sucC* and *sucD* to increase L-lysine production. In addition, the prior art discusses increasing the activity of acetohydroxy acid synthase to increase the production of valine biosynthesis in Corynebacterium glutamicum (Ruklisha et al. 2007).

An aconitase is an enzyme of the tricarboxylic acid cycle, which catalyzes two-step chemical reactions: the transformation of citric acid to aconitic acid and the transformation of aconitic acid of isocitric acid. In the known bacteria of *Escherichia*, the gene *acnA*, the nucleotide sequence of which is shown in SEQ ID No: 1, encodes aconitase A. However, perhaps due to its metabolite far away from the final product L-lysine as well as many and complex intermediary metabolism branches, its role in the L-lysine fermentation has not been suggested. And there are no reports about the effects of an aconitase from other L-lysine-producing bacteria such as *Corynebacterium glutamicum* and the like on the L-lysine fermentation. The aconitases described in documents such as Chinese patents CN1289368A and CN101631871A relate to the fermentation of L-glutamic acid, and are not taught to have any effects on the fermentation of L-lysine.

After a long research and practice, especially by virtue of some luck, the inventors fortuitously found that a modification of an aconitase gene and regulatory element thereof is conducive to the increase of an L-lysine yield.

In addition, in prior arts, an expression amount and/or enzyme activity are increased by the increase of copy number and the introduction of site-directed mutation of a useful enzyme gene, or an enzyme activity and/or expression amount are eliminated by the knockout of a harmful enzyme gene. However the inventors found that an aconitase gene and regulatory element thereof are different from those in prior arts and can not be simply increased or knocked out. Especially the elimination of the expression of the gene *acnA* by the knockout results in slow growth of bacteria and difficulty for a practical use, so a novel method of modifying an aconitase gene and/or regulatory element thereof for increasing an L-lysine yield was invented.

Furthermore, the method does not interfere in modification sites in chromosomes of a great amount of L-lysine-producing bacteria modified according to prior arts, so as to further increase the effect and be useful for practically producing L-lysine by using a great variety of bacteria.

### Disclosure of the Invention

The problem to be solved by the invention is to provide a novel method of producing L-lysine by fermentation and related methods including a method of increasing a fermentation yield of L-lysine compared to an unmodified bacterium, a use of a modified bacterium for producing L-lysine by fermentation, a use of a modified bacterium for increasing a fermentation yield of L-lysine compared to an unmodified bacterium, and/or a method of modifying a bacterium. Furthermore, the disclosure also provides a polynucleotide, vector and/or bacterium used in the methods mentioned above.

Specifically, in first aspect, the invention provides a method of producing L-lysine by fermentation, which comprises the steps of:
(1) modifying an aconitase gene and/or regulatory element thereof in a chromosome of a bacterium so that the activity and/or the expression amount of the aconitase of the bacterium are reduced but not eliminated; and
(2) producing L-lysine by fermentation with the bacterium obtained by the modification of step (1),
wherein modifying the aconitase gene and/or regulatory element thereof includes:
(i) a substitution of GTG for the initiation codon of the nucleotide sequence of SEQ ID NO: 1 of the aconitase gene in the chromosome of an L-lysine producing *Escherichia coli* to GTG;
(ii) a substitution of the nucleotide sequence of SEQ ID NO: 4 of a promoter of the aconitase gene in the chromosome of an L-lysine producing *Escherichia coli* to the nucleotide sequence of SEQ ID NO: 3;
(iii) a substitution of the nucleotide sequence of SEQ ID NO: 6 of a promoter of the aconitase gene in the chromosome of an L-lysine-producing *Corynebacterium glutamicum* or *Corynebacterium pekinese* to the nucleotide sequence of SEQ ID NO: 5;
(iv) a deletion of 90 nucleotides before the termination codon in the nucleotide sequence of the aconitase gene of SEQ ID NO: 1 in the chromosome of an L-lysine producing *Escherichia coli* or;
(v) an addition in the nucleotide sequence of a transcription repressor (SEQ ID NO: 7) of the aconitase gene, preferably an addition of the nucleotide sequence shown in SEQ ID No: 8 and 7 in tandem.

The term "modification" used herein means a change of an object in need of modification resulting in a certain effect. Modification techniques of a gene or a regulatory element in a chromosome include, but are not limited to, mutagenesis, site-directed mutation and/or homologous recombination, preferably the last two. To modify a gene or a regulatory element in a chromosome is an addition, deletion or substitution of one or more nucleotides in the nucleotide sequence of the gene or the regulatory element. Theses techniques are widely described in documents of molecular biology and microbiology, and many of them have been already commercially available. In the embodiments of the disclosure, the modification can be carried out according to the principle of homologous recombination by using the pKOV plasmid commercially available by Addgene or using the pK18mob*sacB* plasmid, so that an unmodified aconitase gene and/or regulatory element thereof in a bacterial chromosome are modified to be a novel aconitase gene and/or regulatory element thereof, and the expression amount and/or the enzyme activity of the aconitase in the modified bacterium are reduced but not eliminated. Therefore preferably the modification used herein is a modification by homologous recombination.

After a long research, the inventors found that in *Escherichia coli,* bacteria of *Corynebacterium* and the like, the elimination of the expression of an aconitase, by using the technique such as the knockout of a gene, results in slow growth of bacteria and even no amino acids produced. Therefore the modification of the invention does reduce and does not eliminate the expression amount of the aconitase of the bacterium obtained by the modification compared to the bacterium without the modification. Preferably the expression amount of aconitase A of the bacterium obtained by the modification is reduced by 20%∼95%, more preferably by 50%∼90%, e.g. by 65%, 70% or 80%.

Accordingly, the disclosure also provides other uses or methods. For example, in second aspect, the disclosure provides a method of increasing a fermentation yield of L-lysine, which comprises the steps of:
(1) modifying an aconitase gene and/or regulatory element thereof in a chromosome of a bacterium so that the activity and/or the expression amount of the aconitase of the bacterium are reduced but not eliminated; and
(2) producing L-lysine by fermentation with the bacterium obtained by the modification of step (1).

L-lysine is an important metabolite of a bacterium, and most of bacteria can generate more or less amount of L-lysine. Although an L-lysine-producing bacterium with low yield is not suitable for cost-efficiently producing L-lysine, yet the methods of the invention can result in the increase of L-lysine and can be used in a cost-insensitive area. Undoubtedly a preferable bacterium used herein is an L-lysine-producing bacterium with high yield, and its yield can be further increased by using the methods of the invention. In addition, in the methods or uses of the invention, except a modification of an aconitase gene and/or regulatory element thereof in a chromosome of a bacterium, no other modifications may be carried out. For example, an aconitase gene in a bacterial chromosome may not be modified while a regulatory element of the aconitase gene is modified, and vice versa. For example, only one of an aconitase gene and regulatory element thereof in a chromosome of a bacterium, especially an L-lysine-producing bacterium with high yield, may be modified.

For example, in third aspect, the disclosure provides a use of a bacterium obtained by a modification for producing L-lysine by fermentation, wherein the modification is to modify an aconitase gene and/or regulatory element thereof in a chromosome of a bacterium, and the enzyme activity and/or the expression amount of the aconitase of the bacterium obtained by the modification are reduced but not eliminated.

The bacterium obtained by the modification may be used alone, with other L-lysine-producing bacteria, or by other means for producing L-lysine by fermentation. Unless otherwise defined (e.g. without the definition of "obtained by the modification"), the term "bacterium" used herein means an unmodified bacterium or a bacterium without the modification, an aconitase gene and a regulatory element located around the locus of the gene in a chromosome of which are an aconitase gene and a regulatory element without the decrease of the enzyme activity and/or the expression amount of the aconitase, e.g., an aconitase gene and a regulatory element from a wild-type bacterium.

For example, in fourth aspect, the disclosure provides a use of a bacterium obtained by a modification for increasing a fermentation yield of L-lysine, wherein the modification is to modify an aconitase gene and/or regulatory element thereof in a chromosome of a bacterium, and the enzyme activity and/or the expression amount of the aconitase of the bacterium obtained by the modification are reduced but not eliminated..

As used herein, a bacterium may be an L-lysine producing bacterium, e.g. a bacterium of *Escherichia* or *Corynebacterium.* In one preferable aspect, a bacterium may be a bacterium of *Escherichia*, more preferably *Escherichia coli,* e.g. filial strains of *Escherichia coli* K-12 including strains derived from the strain W3110; In the other preferable aspect, a bacterium may be a bacterium of *Corynebacterium,* more preferably *Corynebacterium glutamicum* or *Corynebacterium pekinense.* Because few prior arts suggest the role of an aconitase gene and/or regulatory element thereof from a bacterium in the production/fermentation of L-lysine and focus on the loci such as *pnt*, *dap*, *ppc* and the like to modify the genes in chromosomes, there are no reports about a modification of an aconitase gene and/or regulatory element therearound from L-lysine-producing bacteria (especially bacteria of *Escherichia* or *Corynebacterium,* e.g. *E. coli*, *Corynebacterium glutamicum* or *Corynebacterium pekinense*) in prior arts, and thus the L-lysine-producing bacteria substantially have wild-type aconitase genes and regulatory elements therearound. So they may be modified by using the methods of the invention for the increase of a fermentation yield of L-lysine. In the embodiments of the invention, all of L-lysine-producing bacteria with low or high yield can be modified by using the methods of the invention to increase a fermentation yield of L-lysine.

Substantially, in fifth aspect, the disclosure provides a method of modifying a bacterium, which comprises the step of modifying an aconitase gene and/or regulatory element thereof in a chromosome of a bacterium so that the activity and/or the expression amount of the aconitase of the bacterium obtained by the modification are reduced but not eliminated.

The bacterium obtained by the method of the fifth aspect of the disclosure can be used for producing or generating L-lysine by fermentation. Therefore in sixth aspect, the disclosure provides a bacterium obtained by the method of the fifth aspect of the disclosure.

According to the experiments by the inventors, many of nucleotide sequences of aconitase genes (*acnA*) from bacteria of *Escherichia* (e.g., *E. coli*) are shown in SEQ ID No: 1, while many of nucleotide sequences of aconitase genes from bacteria of *Corynebacterium* (e.g., *Corynebacterium glutamicum* or *Corynebacterium pekinense*) are shown in SEQ ID No: 2. To modify these aconitase genes so as to reduce but not eliminate the enzyme activity and/or the expression of the aconitases in the modified bacteria, can increase a fermentation yield of L-lysine. Therefore in the invention, the nucleotide sequence of the aconitase gene is shown in SEQ ID No: 1 or 2. For other bacteria, the nucleotide sequences of aconitase genes from the bacteria can be obtained by the techniques such as sequencing and comparing identities of sequences for the modification of the methods of the invention.

In the disclosure, the preferable step of modifying an aconitase gene in a chromosome of a bacterium is an addition, deletion or substitution of one or more nucleotides in the nucleotide sequence of the aconitase gene, as long as the enzyme activity and/or the expression amount of the aconitase from the modified bacterium are reduced but not eliminated.

According to the experience of the inventors, although it is usually difficult to increase an activity and/or an expression amount of a bacterium's enzyme since the bacterium has been evolved for a long time and modification sites for the increase need be researched particularly, yet the research for the decrease of the activity and/or the expression amount of the enzyme is much easier. For example, the nucleotide sequence out of domains of an enzyme may be mutated. In the disclosure, the preferable step of modifying an aconitase gene in a chromosome of a bacterium may be a substitution of one or more nucleotides in the nucleotide sequence of the aconitase gene. For the invention the substitution is for the initiation codon of the aconitase geneand is a substitution of GTG. It can be carried out for *Escherichia coli.* Also in the disclosure, the preferable step of modifying an aconitase gene in a chromosome of a bacterium may be a deletion of one or more nucleotides in the nucleotide sequence of the aconitase gene. For the invention, the deletion is a deletion of 90 nucleotides before the termination codon in the nucleotide sequence of the aconitase gene. It can be carried out for *Escherichia coli.*

As used herein, a regulatory element means a polynucleotide located upstream or downstream of a gene (e.g., an aconitase gene) and regulating the transcription and/or the expression of the gene in order to have an effect on the expression amount of the gene. It may include non-coding and encoding sequences. A regulatory element may be a promoter, enhancer, repressor or other polynucleotide related to transcription and/or expression control. A preferable regulatory element may be a promoter. In the invention, the nucleotide sequence of the promoter is shown in SEQ ID No: 4 or 6. A preferable regulatory element may also be a repressor, for example a transcription repressor. In the invention, the nucleotide sequence of the transcription repressor is shown in SEQ ID No: 7. By modifying a promoter and/or repressor, an enzyme activity and/or expression amount of an aconitase in a modified bacterium are reduced but not eliminated.

In the disclosure, the preferable step of modifying a regulatory element of an aconitase gene in a chromosome of a bacterium is an addition, deletion or substitution of one or more nucleotides in the nucleotide sequence of the regulatory element of the aconitase gene, as long as the enzyme activity and/or the expression amount of the aconitase from the modified bacterium are reduced but not eliminated.

According to the experience of the inventors, although it is usually difficult to increase a transcription activity of a bacterium's promoter or enhancer since the bacterium has been evolved for a long time and modification sites for the increase need be researched particularly, yet the research for the decrease of the transcription activity of the promoter or enhancer is much easier. For example, one or several nucleotide sequence can be added for increasing the distance between a promoter and the initiation codon of a gene, or a promoter with weak transcription activity is substituted for an original promoter. In the disclosure, the preferable step of modifying a regulatory element of an aconitase gene in a chromosome of a bacterium may be a substitution of one or more nucleotides in a promoter of the aconitase gene. For the invention, the substitution of a promoter with weak transcription activity is a substitution of the nucleotide sequence shown in SEQ ID No: 3 or 5.

Furthermore, in the disclosure, the preferable step of modifying a regulatory element of an aconitase gene in a chromosome of a bacterium may be an addition of the nucleotide sequence of a transcription repressor for the aconitase gene, for example an addition of the nucleotide sequence of a new transcription repressor or increase of the copy number of the nucleotide sequence of an original transcription repressor. A transcription repressor may be added behind a promoter (especially a promoter with strong transcription activity) in order to increase its expression amount and inhibit the transcription of an aconitase gene more efficiently. In the invention, the nucleotide sequence shown in SEQ ID No: 8 and 7 in tandem is added.

Furthermore, the disclosure also provides intermediate products such as a polynucleotide and/or vector used in the methods mentioned above. For example, in seventh aspect, the disclosure provides a polynucleotide, the nucleotide sequence of which is selected from
(a) the nucleotide sequence obtained by a substitution (preferably of GTG) for the initiation codon of the nucleotide sequence shown in SEQ ID No: 1;
(b) the nucleotide sequence obtained by a deletion (preferably of 1-120 nucleotides, more preferably of 1-90 nucleotides, most preferably of 90 nucleotides) in the nucleotide sequence shown in SEQ ID No: 1 or 2, e.g. a deletion of 90 nucleotides before the termination codon in the nucleotide sequence shown in SEQ ID No: 1 or 2; and
(c) the nucleotide sequence shown in SEQ ID No: 8 or 7 in tandem.

In eighth aspect, the disclosure provides a vector, which comprises the polynucleotide of the seventh aspect of the disclosure.

In ninth aspect, the disclosure provides a use of the polynucleotide of the seventh aspect of the disclosure and/or the vector of the eighth aspect of the disclosure in the method or use of the first, second, third and/or fourth aspect of the disclosure. That is that in the methods or uses of the first, second, third and/or fourth aspects of the disclosure, the polynucleotide of the seventh aspect of the disclosure and/or the vector of the eighth aspect of the disclosure are used.

In tenth aspect, the disclosure provides a use of the polynucleotide of the seventh aspect of the disclosure and/or the vector of the eighth aspect of the disclosure in the preparation of the bacterium of the fifth aspect of the disclosure. That is that in the preparation process of the bacterium of the fifth aspect of the disclosure, the polynucleotide of the seventh aspect of the disclosure and/or the vector of the eighth aspect of the disclosure are used.

The beneficial effects of the disclosure include that novel techniques are invented and proved for increasing a fermentation yield of L-lysine when using L-lysine-producing bacteria with high and low yield; and do not interfere in modification sites in chromosomes of a great amount of L-lysine-producing bacteria with high yield modified according to prior arts, so as to further increase the yield, be useful for practically producing L-lysine by the fermentation of bacteria, and be easy to popularize.

For a better understanding of the invention, it will now be described in greater detail by reference to specific examples. It should be noted that the examples only exemplify the invention. According to the description of the invention, various modifications and alterations of the invention are obvious to a person skilled in the art.

### Examples

Embodiments of the invention are further exemplified by the following examples. Unless otherwise specified, the techniques used in the examples are well-known for a person skilled in the art and commercially available devices and reagents (see Molecular Cloning: A Laboratory Manual (3rd Edition), Science Press, Microbiology Experiments (4th Edition), Higher Education Press, and the manufacturer's instructions of the devices and reagents).

### Example 1. Substitution of GTG for the initiation codon ATG of aconitase

The genomic chromosome of the wild-type *Escherichia coli* strain, *E. coli* K12 W3110 (commercially available from Biological Resource Center, National Institute of Technology and Evaluation (NITE Biological Resource Center, NBRC)), was extracted as a template for a PCR amplification, by using the primers P1/P2 and P3/P4 respectively. Two DNA fragments of 510bp and 620bp were obtained and named Up1 and Down1 fragments respectively. The process of the PCR amplification was shown as follows: denaturalizing for 30s (seconds) in 94°C, annealing for 30s (seconds) in 52°C, extending for 30s (seconds) in 72°C, and performing for 30 times circularly. And the sequences of the primers were shown as follows:
P1: 5'-CGCGGATCCGGAGTCGTCACCATTATGCC-3'
P2: 5'-TCTCGTAGGGTTGACGACACAGCTCCTCCTTAATGACAGG-3'
P3: 5'-CCTGTCATTAAGGAGGAGCTGTGTCGTCAACCCTACGAGA-3'
P4: 5'-ATTGCGGCCGCTCCATTCACCGTCCTGCAAT-3'

The two DNA fragments were purified by agarose gel electrophoresis and mixed as a template for overlap PCR amplification by using the primers P1/P4. An approximately 1200bp fragment was obtained and named Up-Down1 fragment. The process of the PCR amplification was shown as follows: denaturalizing for 30s (seconds) in 94°C, annealing for 30s (seconds) in 52°C, extending for 60s (seconds) in 72°C, and performing for 30 times circularly.

The Up-Down1 purified by agarose gel electrophoresis and the pKOV plasmid (commercially available from Addgene) were digested respectively by *Bam* HI/*Not* I. The digestion products were purified by agarose gel electrophoresis and ligated to be a vector pKOV-Up-Down1 for further transform. The vector pKOV-Up-Down1 was determined by sequencing the vector by a sequencing company to have a correct point mutation (A-G) in the gene fragment of *acnA*, and stored for further use.

The constructed plasmid pKOV-Up-Down1 was transformed by electroporation into the L-lysine-producing strain with low yield, *E. coli* NRRL B-12185 (commercially available from Agricultural Research Service Culture Collection (NRRL); see also US4346170A for its construction method), and the L-lysine-producing strain with high yield, *E. coli* K12 W3110 Δ3 (commercially available from Institute of Microbiology, Chinese Academy of Sciences; the strain is an L-lysine-producing engineering strain mutagenized and mutated from *E. coli* K12 W3110) respectively. The two strains were determined by sequencing to have the wild-type gene of *acnA* (i.e., sites 1333855 to 1336530 of GenBank accession number U00096.2) and upstream and downstream elements thereof in their chromosomes. According to the manufacturer's instruction of pKOV plasmid from Addgene, homologous recombination-positive clones were selected after a recovery culture in LB medium under the conditions of 30°C and 100 rpm for 2h, and were determined by sequencing to have the mutation of the initiation codon ATG to GTG in the wild-type gene of *acnA* in their chromosomes. Finally both of L-lysine-producing *E. coli* strains with low and high yield having the mutation of the initiation codon of *acnA* were obtained and named YP-13633 and YP-13664 respectively. The expression amount of aconitases in the two modified strains was measured and reduced by approximately 75∼85% (different values in different media).

### Example 2. Mutation of the aconitase gene sequence for the decrease of the activity of aconitase

### (1) Construction of E. coli strains

90 nucleotides before the termination codon in the gene *acnA* of *E. coli* were deleted for the decrease of the aconitase activity. Specifically, the genomic chromosome of the wild-type *Escherichia coli* strain, *E. coli* K12 W3110, was extracted as a template for PCR amplification, by using the primers P5/P6 and P7/P8 respectively. Two DNA fragments of 752bp and 657bp were obtained and named Up2 and Down2 fragments respectively. The process of the PCR amplification was shown as follows: denaturalizing for 30s (seconds) in 94° C, annealing for 30s (seconds) in 52° C, extending for 30s (seconds) in 72° C, and performing for 30 times circularly. And the sequences of the primers were shown as follows:
P5: 5'-CGCGGATCCCGTCACACGATCCGATACCT-3'
P6: 5'-CGGCAAGCAAATAGTTGTTATACGACTTCCTGGCTACCAT -3'
P7: 5'-ATGGTAGCCAGGAAGTCGTATAACAACTATTTGCTTGCCG-3'
P8: 5'-ATTGCGGCCGC CATGGGGCGATTTCCTGATG-3'

The two DNA fragments were purified by agarose gel electrophoresis and mixed as a template for an overlap PCR amplification by using the primers P5/P8. An approximately 1400bp fragment was obtained and named Up-Down2 fragment. The process of the PCR amplification was shown as follows: denaturalizing for 30s (seconds) in 94° C, annealing for 30s (seconds) in 52° C, extending for 60s (seconds) in 72° C, and performing for 30 times circularly.

The Up-Down2 purified by agarose gel electrophoresis and the pKOV plasmid (commercially available from Addgene) were digested respectively by *Bam* HI/*Not* I. The digestion products were purified by agarose gel electrophoresis and ligated to be a vector pKOV-Up-Down2 for further transform. The vector pKOV-Up-Down2 was determined by sequencing the vector by a sequencing company to have a deletion of 90bp bases before the termination codon in the gene fragment of *acnA*, and stored for further use.

According to the manufacturer's instruction of pKOV plasmid from Addgene, the constructed plasmid pKOV-Up-Down2 was transformed by electroporation into the L-lysine-producing strain with low yield, *E. coli* NRRL B-12185 (see also US4346170A for its construction method), and the L-lysine-producing strain with high yield, *E. coli* K12 W3110 Δ3 respectively. The two strains were determined by sequencing to have the wild-type gene of *acnA* and upstream and downstream elements thereof in their chromosomes. Homologous recombination-positive clones were selected, and were determined by sequencing to have the deletion of 90bp bases before the termination codon in the gene of *acnA* in their chromosomes. Finally both of L-lysine-producing *E. coli* strains with low and high yield, in which the enzyme activities of *acnA* were reduced, were obtained and named YP-13675 and YP-13699 respectively. The aconitase activities in the two modified strains were measured and reduced by approximately 60∼80% (different values in different media).

### (2) Construction of Corynebacterium strains

90 nucleotides before the termination codon in the *acn* gene of *Corynebacterium* were deleted for the decrease of the aconitase activity. The process of construction was substantially the same as that of step (1) mentioned above, except that the genome of *Corynebacterium pekinense* AS 1.299 (which is similar to *Corynebacterium glutamicum* and is sometimes mistakenly regarded as *Corynebacterium glutamicum*; commercially available from China General Microbiological Culture Collection Center (CGMCC)) was used as a template for an amplification by using the primers P9∼P12 as follows (corresponding to the primers P4∼P8 mentioned above respectively), and a 542bp fragment (Up2), a 527bp fragment (Down2), and an approximately 1069bp fragment (Up-Down) were obtained. And the sequences of the primers were shown as follows:
P9: 5'-CGGGATCCTGCAGCTCAGTACTTGGAT-3'
P10: 5'-AAAGTCTTCTAATTAC TTACTGCGTCGAACTCGACG-3'
P11: 5'-GTTCGACGCAGTAAGTAATTAGAAGACTTTTGAT-3'
P12: 5'-TCCCCCGGGGAATACCGGGTCGGTGCG-3'

Then the Up-Down2 fragment purified by agarose gel electrophoresis and pK18mob*sacB* plasmid (commercially available from American Type Culture Collection (ATCC)) were digested respectively by *Bam* HI/*Sma* I. The digestion products were purified by agarose gel electrophoresis and ligated to be a recombinant vector pK18mob*sacB*-Up-P-Down having a deletion of 90bp bases before the termination codon in the *acn* gene. The constructed plasmid pK18mob*sacB*-Up-Down2 was verified by sequencing and transformed respectively by electroporation into the L-lysine-producing strain with low yield, *Corynebacterium pekinense* AS 1.299, the L-lysine-producing strain with low yield, *Corynebacterium glutamicum* ATCC 13032 (commercially available from ATCC), and the L-lysine-producing strain with high yield, *Corynebacterium pekinense* AS 1.563 (commercially available from China General Microbiological Culture Collection Center). The last two strains were determined by sequencing to have in their chromosomes the *acn* gene from *Corynebacterium pekinense* AS 1.299, the nucleotide sequence of which is shown in SEQ ID NO: 2. Homologous recombination-positive clones were selected after a recovery culture in BHIS medium under the conditions of 30° C and 120 rpm for 2h, and were verified by sequencing. Finally three L-lysine-producing *Corynebacterium* strains with low, low and high yield having the mutation of the *acn* gene were obtained and named YP-14808, YP-14852 and YP-14837.

### Example 3. Substitution of a promoter with weak transcription activity for the promoter of aconitase gene

### (1) Construction of E. coli strains

By the analysis of the upstream sequence of *acnA* in *E. coli* K12 W3110, we provided a promoter with weak transcription activity (the sequence of which is shown in SEQ ID No: 3) to replace the region of the wild-type promoter (the sequence of which is shown in SEQ ID No: 4) in the upstream of the ORF of the *acnA* gene for the decrease of the expression of the wild-type *acnA* gene.

Specifically, the genomic chromosome of the wild-type *Escherichia coli* strain, *E. coli* K12 W3110, was extracted as a template for PCR amplification, by using the primers P13/P14 and P15/P16 respectively. Two DNA fragments of 580bp and 618bp were obtained and named Up3 and Down3 fragments respectively. A plasmid comprising the promoter with weak transcription activity mentioned above was used as a template for PCR amplification by using P17/P18, and a 161bp promoter fragment with weak transcription activity was obtained and named P fragment. The process of the PCR amplification was shown as follows: denaturalizing for 30s (seconds) in 94° C, annealing for 30s (seconds) in 52° C, extending for 30s (seconds) in 72° C, and performing for 30 times circularly.

The three DNA fragments mentioned above were purified by agarose gel electrophoresis. Then the Up3 and P fragments were mixed as a template for overlap PCR amplification by using the primers P13/P18. An approximately 716bp fragment was obtained and named Up-P fragment. The process of the PCR amplification was shown as follows: denaturalizing for 30s (seconds) in 94° C, annealing for 30s (seconds) in 52° C, extending for 60s (seconds) in 72° C, and performing for 30 times circularly.

The Up-P and Down3 fragments purified by agarose gel electrophoresis were mixed as a template for an overlap PCR amplification by using the primers P13/P16. An approximately 1334bp fragment was obtained and named Up-P-down fragment. The process of the PCR amplification was shown as follows: denaturalizing for 30s (seconds) in 94° C, annealing for 30s (seconds) in 52° C, extending for 60s (seconds)) in 72° C, and performing for 30 times circularly.

The sequences of the primers mentioned above were shown as follows:
P13: 5'-CGCGGATCCGAAGAAATTGAGGTCATGTT -3'
P14: 5'- GGTTTCTTAGACGTCGGATTCGTTTCGTTTCTGTTTCATT-3'
P15: 5'- ATCAGCAGGACGCACTGACCCATTAAGGAGGAGCTATGTCG -3'
P16: 5'- ATTGCGGCCGCTCCATTCACCGTCCTGCAATT -3'
P17: 5'- AATGAAACAGAAACGAAACGCAATCCGACGTCTAAGAAACC -3'
P18: 5'- CGACATAGCTCCTCCTTAATGGGTCAGTGCGTCCTGCTGAT -3'

The Up-P-down purified by agarose gel electrophoresis and the pKOV plasmid (commercially available from Addgene) were digested respectively by *Bam* HI/*Not* I . The digestion products were purified by agarose gel electrophoresis and ligated to be a vector pKOV-Up-P-Down for further transform. The vector pKOV-Up-P-Down was determined by sequencing the vector by a sequencing company to have a correct sequence of the promoter with weak transcription activity, and stored for further use.

The constructed plasmid pKOV-Up-P-Down was transformed by electroporation into the L-lysine-producing strain with low yield, *E. coli* NRRL B-12185, and the L-lysine-producing strain with high yield, *E. coli* K12 W3110 Δ3 respectively. The two strains were determined by sequencing to have the wild-type gene of *acnA* and upstream and downstream elements thereof in their chromosomes, where the upstream promoter comprises sites form 2102518 to 2102713 of GenBank accession number CP004009.1. According to the manufacturer's instruction of pKOV plasmid from Addgene, homologous recombination-positive clones were selected after a recovery culture in LB medium under the conditions of 30° C and 100 rpm for 2h, and were determined by sequencing to have the substitution of the promoter with weak transcription activity for the wild-type promoter in the upstream of the gene *acnA* in their chromosomes. Finally both of L-lysine-producing *E. coli* strains with low and high yield having the mutation of the promoter of *acnA* were obtained and named YP-13627 and YP-13682 respectively. The expression amount of aconitases in the two modified strains was measured and reduced by 65∼80% in different media.

### (2) Construction of Corynebacterium strains

By the analysis of the upstream sequence of *acn* in *Corynebacterium,* we provided a promoter with weak transcription activity (the sequence of which is shown in SEQ ID No: 5) to replace the region of the 166 bp wild-type promoter (the sequence of which is shown in SEQ ID No: 6) in the upstream of the ORF of the *acn* gene from *Corynebacterium.* The process of construction was substantially the same as that of step (1) mentioned above, except that:

The genome of *Corynebacterium pekinense* AS 1.299 (commercially available from China General Microbiological Culture Collection Center) was used as a template for an amplification by using the primers P19∼P24 as follows (corresponding to the primers P13∼P18 mentioned above respectively), and a 573bp fragment (Up3), a 581bp fragment (Down3), a 130bp fragment (P), and an approximately 1284bp fragment (Up-P-down) were obtained. And the sequences of the primers were shown as follows;
P19: 5'-CGGGATCCGCCAAAGCAACCAACCCC -3'
P20: 5'-CTTTTTAGTTTTCAACGGTCGGATTTGCTCGAAAT-3'
P21: 5'- GCCGAAAC AAAGTAGCCGAAGCAGACGCCGTCG -3'
P22: 5'-CGGAATTCTGACCTGGTGGACGATAC-3'
P23: 5'-CGAGCAAATCCGACCGTTGAAAACTAAAAAGCTGG-3'
P24: 5'-GCGTCTGCTTCGGCTACTTTGTTTCGGCCACCC-3'

Then the Up-P-down fragment purified by agarose gel electrophoresis and the pK18mo*bsacB* plasmid were digested respectively by *Bam* HI*lEco* RI. The digestion products were purified by agarose gel electrophoresis and ligated to be a recombinant vector pK18mob*sacB*-Up-P-Down for further substitution of the promoter. The constructed plasmidpK18mob*sacB*-Up-P-Down was verified by sequencing and transformed respectively by electroporation into the L-lysine-producing strain with low yield, *Corynebacterium pekinense* AS 1.299, the L-lysine-producing strain with low yield, *Corynebacterium glutamicum* ATCC 13032, and the L-lysine-producing strain with high yield, *Corynebacterium pekinense* AS 1.563. The last two strains were determined by sequencing to have in their chromosomes the *acn* gene and upstream and downstream elements thereof from *Corynebacterium pekinense* AS 1.299. Homologous recombination-positive clones were selected after a recovery culture in BHIS medium under the conditions of 30°C and 120 rpm for 2h, and were verified by sequencing. Finally three L-lysine-producing *Corynebacterium* strains with low, low and high yield having the mutation of the promoter of *acn* were obtained and named YP-14755, YP-14732 and YP-14780.

### Example 4. Increase of the copy number of the gene acnR encoding the transcription repressor of aconitase gene

The copy number of the gene *acnR* encoding the transcription repressor of *acn* was increased for the decrease of the transcription level of the *acn* gene. Specifically, the genome of *Corynebacterium pekinense* AS 1.299 was used as a template for PCR amplification by using the primers P25/P26 and P27/P28 respectively. Two DNA fragments of 715bp and 797bp were obtained and named Up4 and Down4 fragments. The template was used or a PCR amplification by using the primers P29/P30. A 567 bp *acnR* fragment was obtained and named R fragment, the nucleotide sequence of which is shown in SEQ ID No: 7. In addition, the expression plasmid pXMJ19 (commercially available from Biovector Science Lab, Inc) was used as a template for a PCR amplification by using the primers P31 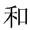 P32. An 164bp promoter P*_{tac}* with strong transcription activity was obtained and named P*_{tac}* fragment, the nucleotide sequence of which is shown in SEQ ID No: 8. The process of the PCR amplification was shown as follows: denaturalizing for 30s (seconds) in 94°C, annealing for 30s (seconds) in 52°C, extending for 30s (seconds) in 72°C, and performing for 30 times circularly.

The sequences of the primers mentioned above were shown as follows:
P25: 5'-CGGGATCCTTCGCAACCGATAGAGCA-3'
P26: 5'-CACGAATTATGCAGAATAAGCCTTTAAGTAACAA-3'
P27: 5'-TAAACGCGACTAAGCGTGACCATTAAAAGGCT-3'
P28: 5'-CGGAATTCAAAAGCCTATTAAGTGTC-3'
P29: 5'-TTCACACAGGAAAGTGTCCGTAGCGGCAGGCGA-3'
P30: 5'-TTTAATGGTCACGC TTAGTCGCGTTTACGGACAG-3'
P31: 5'-TTAAAGGCTTATTCTGCATAATTCGTGTCGCTC-3'
P32: 5'-GCCGCTACGGACACTTTCCTGTGTGAAATTGTTA-3'

The R and P*_{tac}* fragments were purified by agarose gel electrophoresis and mixed as a template for an overlap PCR amplification by using the primers P31/P30. An approximately 731bp fragment was obtained and named P*_{tac}*-R fragment. The Up4 and P*_{tac}*-R fragments were purified by agarose gel electrophoresis and mixed as a template for overlap PCR amplification by using the primers P25/P30. An approximately 1446bp fragment was obtained and named Up4-P*_{tac}*-R fragment. The Up4-P*_{tac}*-R and Down4 fragments were purified by agarose gel electrophoresis and mixed as a template for an overlap PCR amplification by using the primers P25/P28. An approximately 2243bp fragment was obtained and named Up-P*_{tac}*-R-Down fragment. The process of the PCR amplification was shown as follows: denaturalizing for 30s (seconds) in 94°C, annealing for 30s (seconds) in 52°C, extending for 60s (seconds) in 72°C, and performing for 30 times circularly.

Then the Up-P*_{tac}*-R-Down purified by agarose gel electrophoresis and the pK18mob*sacB* plasmid were digested respectively by *Bam* HI/*Eco* RI. The digestion products were purified by agarose gel electrophoresis and ligated to be a recombinant vector pK18mob*sacB*-Up-P*_{tac}*-R-Down for further insertion of additional copies of *acnR* into a non-coding region of a chromosome. The vector pK18mob*sacB*-Up-P*_{tac}*-R-Down was determined by sequencing the vector by a sequencing company to have the *P_{tac}-acnR* gene fragments, and stored for further use.

The constructed plasmid pK18mobsacB-Up-P*_{tac}*-R-Down was transformed respectively by electroporation into the L-lysine-producing strain with low yield, *Corynebacterium pekinense* AS 1.299, the L-lysine-producing strain with low yield, *Corynebacterium glutamicum* ATCC 13032, and the L-lysine-producing strain with high yield, *Corynebacterium pekinense* AS1.563. Homologous recombination-positive clones were selected after a recovery culture in BHIS medium under the conditions of 30°C and 100 rpm for 2h, and were verified by sequencing. Finally three L-lysine-producing *Corynebacterium* strains with low, low and high yield, into non-coding regions of the chromosomes of which additional copies of the gene *acnR* were inserted, were obtained and named YP-14857, YP-14896 and YP-14860.

### Example 5. L-lysine fermentation experiments

For the fermentation of *E. coli,* 25mL of the seed medium shown in table 1 was inoculated with each of the strains *E. coli* K12 W3110 Δ3, *E. coli* NRRL B-12185 and *E. coli* strains constructed by examples 1∼3 respectively, and cultured under the conditions of 37°C and 220 rpm for 9 h. Then 25mL of the fermentation medium shown in table 1 was inoculated with 1mL culture product of the seed medium, and cultured under the conditions of 37°C and 220 rpm for 48 h. The yield of L-lysine was measured by HPLC after the culture process.

**Table 1 Culture medium for E. coli**

| | Seed medium | Fermentation medium |
|---|---|---|
| (Ingredient) | (g/L) | (g/L) |
| glucose | 15 | 40 |
| ammonium sulphate | 4 | 10 |
| potassium dihydrogen phosphate | 3 | 1.6 |
| magnesium sulphate heptahydrate | 0.4 | 1 |
| ferrous sulphate heptahydrate | 0.01 | 0.03 |
| manganese sulfate monohydrate | 0.01 | 0.03 |
| yeast extract | 2.0 | 4.0 |
| calcium carbonate | | 25 |
| KOH | pH 7.0 | pH 7.0 |
| L-tyrosine | | 0.1 |
| L-methionine | | 0.5 |
| L-threonine | | 0.1 |
| L-isoleucine | | 0.05 |

For the fermentation of *Corynebacterium,* 30mL of the seed medium shown in table 2 was inoculated with each of the strains *Corynebacterium* AS 1.299, ATCC13032, AS1.563 and *Corynebacterium* strains constructed by examples 2∼4 respectively, and cultured under the conditions of 30°C and 220 rpm for 8h. Then 30mL of the fermentation medium shown in table 2 was inoculated with 1mL culture product of the seed medium, and cultured under the conditions of 30°C and 220 rpm for 48 h. The yield of L-lysine was measured by HPLC after the culture process.

**Table 2 Culture medium for Corynebacterium**

| | Seed medium | Fermentation medium |
|---|---|---|
| (Ingredient) | (g/L) | (g/L) |
| glucose | 20 | 40 |
| ammonium sulphate | 5 | 20 |
| potassium dihydrogen phosphate | 1 | 1.6 |
| magnesium sulphate heptahydrate | 0.7 | 0.8 |
| ferrous sulphate heptahydrate | 0.01 | 0.01 |
| manganese sulfate monohydrate | 0.01 | 0.01 |
| yeast extract | 5.0 | 4.0 |
| calcium carbonate | | 20 |
| KOH | pH 7.0 | pH 7.0 |

The results are show in table 3. To mutate (e.g., delete or replace) the aconitase structure of the strains of *Escherichia coli* and *Corynebacterium* for the decrease of the activity of the enzyme, or to modify (e.g., replace and insert) the regulatory elements of the aconitase genes of the strains of *Escherichia coli* and *Corynebacterium* for the decrease of the expression amount of the enzyme, is conducive to the increase of L-lysine yield.

**Table 3 Yield of L-Lysine by Strains**

| Strain | Yield of L-lysine (g/L) | Yield increase ratio (%) |
|---|---|---|
| *E. coli* NRRL B-12185 | 1.5 | - |
| YP-13633 | 2.1 | 40 |
| YP-13675 | 1.8 | 20 |
| YP-13627 | 2.0 | 33 |
| *E. coli* K12 W3110 Δ3 | 10.2 | - |
| YP-13664 | 16.1 | 57.8 |
| YP-13699 | 12.5 | 22.5 |
| YP-13682 | 14.1 | 38.2 |
| *Corynebacterium* AS 1.299 | 1.2 | - |
| YP-14808 | 1.6 | 33 |
| YP-14755 | 1.9 | 58 |
| YP-14857 | 1.4 | 17 |
| *Corynebacterium* ATCC 13032 | 1.1 | - |
| YP-14852 | 1.5 | 36 |
| YP-14732 | 2.0 | 82 |
| YP-14896 | 1.3 | 18 |
| *Corynebacterium* AS1.563 | 23.5 | - |
| YP-14837 | 27.4 | 16.6 |
| YP-14780 | 31.2 | 32.8 |
| YP-14860 | 25.6 | 8.9 |

## Claims

1. A method of producing L-lysine by fermentation or of increasing a fermentation yield of L-lysine, which comprises the steps of:
(1) modifying an aconitase gene and/or regulatory element thereof in a chromosome of an L-lysine producing bacterium so that the activity and/or the expression amount of the aconitase of the bacterium are reduced but not eliminated; and
(2) producing L-lysine by fermentation with the bacterium obtained by the modification of step (1),
wherein modifying the aconitase gene and/or regulatory element thereof includes:
(i) a substitution of the initiation codon of the nucleotide sequence of SEQ ID NO: 1 of the aconitase gene in the chromosome of an L-lysine producing *Escherichia coli* to GTG;
(ii) a substitution of the nucleotide sequence of SEQ ID NO: 4 of a promoter of the aconitase gene in the chromosome of an L-lysine producing *Escherichia coli* to the nucleotide sequence of SEQ ID NO: 3;
(iii) a substitution of the nucleotide sequence of SEQ ID NO: 6 of a promoter of the aconitase gene in the chromosome of an L-lysine-producing *Corynebacterium glutamicum* or *Corynebacterium pekinese* to the nucleotide sequence of SEQ ID NO: 5;
(iv) a deletion of 90 nucleotides before the termination codon in the nucleotide sequence of the aconitase gene of SEQ ID NO: 1 in the chromosome of an L-lysine producing *Escherichia coli*; or
(v) an addition in the nucleotide sequence of a transcription repressor (SEQ ID NO: 7) of the aconitase gene, preferably an addition of the nucleotide sequence shown in SEQ ID No: 8 and 7 in tandem.

## Patentansprüche

1. Verfahren zur Herstellung von L-Lysin durch Fermentation oder durch Erhöhen einer Fermentationsausbeute von L-Lysin, welches die folgenden Schritte umfasst:
(1) das Modifizieren eines Aconitasegens und/oder eines regulatorischen Elements davon in einem Chromosom eines L-Lysin produzierenden Bakteriums, so dass die Aktivität und/oder die Expressionsmenge der Aconitase des Bakteriums reduziert, jedoch nicht eliminiert sind, und
(2) das Herstellen von L-Lysin durch Fermentation mit dem durch die Modifikation von Schritt (1) erhaltenen Bakterium,
wobei das Modifizieren des Aconitasegens und/oder des regulatorischen Elements davon Folgendes umfasst:
(i) eine Substitution des Startcodons der Nukleotidsequenz von SEQ ID NO: 1 des Aconitasegens im Chromosom eines L-Lysin produzierenden *Escherichia coli* durch GTG,
(ii) eine Substitution der Nukleotidsequenz von SEQ ID NO: 4 eines Promotors des Aconitasegens im Chromosom eines L-Lysin produzierenden *Escherichia coli* durch die Nukleotidsequenz von SEQ ID NO: 3,
(iii) eine Substitution der Nukleotidsequenz von SEQ ID NO: 6 eines Promotors des Aconitasegens im Chromosom eines L-Lysin produzierenden *Corynebacterium glutamicum* oder *Corynebacterium pekinese* durch die Nukleotidsequenz von SEQ ID NO: 5,
(iv) eine Deletion von 90 Nukleotiden vor dem Stoppcodon in der Nukleotidsequenz des Aconitasegens von SEQ ID NO: 1 im Chromosom eines L-Lysin produzierenden *Escherichia coli* oder
(v) eine Addition in der Nukleotidsequenz eines Transkriptionsrepressors (SEQ ID NO: 7) des Aconitasegens, vorzugsweise eine Addition der in SEQ ID NO: 8 und 7 gezeigten Nukleotidsequenz hintereinander.

## Revendications

1. Procédé de production de L-Lysine par fermentation ou d'augmentation du rendement d'une fermentation de L-Lysine, qui comprend les étapes consistant à :
(1) modifier un gène d'aconitase et/ou un élément de régulation de celui-ci dans un chromosome d'une bactérie productrice de L-Lysine, de sorte que l'activité et/ou la quantité d'expression de l'aconitase de la bactérie soient réduits mais non éliminés ; et
(2) produire de la L-Lysine par fermentation avec la bactérie obtenue par la modification de l'étape (1), dans lequel une modification du gène d'aconitase et/ou d'un élément de régulation de celui-ci comprend :
(i) une substitution du codon d'initiation de la séquence nucléotidique de SEQ ID n° : 1 du gène d'aconitase, dans le chromosome d'un *Escherichia coli* producteur de L-Lysine, en un GTG ;
(ii) une substitution de la séquence nucléotidique de SEQ ID n° : 4 d'un promoteur du gène d'aconitase, dans le chromosome d'un *Escherichia coli* producteur de L-Lysine, en la séquence nucléotidique de SEQ ID n° : 3 ;
(iii) une substitution de la séquence nucléotidique de SEQ ID n° : 6 d'un promoteur du gène d'aconitase, dans le chromosome d'un *Corynebacterium glutamicum* ou *Corynebacterium pekinese* producteur de L-Lysine, en la séquence nucléotidique de SEQ ID n° : 5 ;
(iv) une délétion de 90 nucléotides avant le codon de terminaison dans la séquence nucléotidique du gène d'aconitase de SEQ ID n° : 1, dans le chromosome d'un *Escherichia coli* producteur de L-Lysine ; ou
(v) un ajout, dans la séquence nucléotidique, d'un répresseur de la transcription (SEQ ID n° : 7) du gène d'aconitase, de préférence un ajout de la séquence nucléotidique présentée dans les SEQ ID n° : 8 et 7 en tandem.
